# EUROPEAN PATENT APPLICATION

(11) **EP 1 793 159 A1**
(43) Date of publication of application: **06.06.2007**
(21) Application number: 05026470.4
(22) Date of filing: 05.12.2005
(51) Int. Cl.: F21V 17/00, F21V 29/00, F21V 17/14, A61N 5/06

(54) **Infrared lamp apparatus**

(71) Applicant: Park, Shi-heung, Gwangju-si, Kyeonggi-do Seoul 464-883 (KR)
(72) Inventor: Park, Shi-heung, Gwangju-si, Kyeonggi-do Seoul 464-883 (KR)
(74) Representative: Lohr, Georg

(57) **Abstract**

This invention, concerning an infrared lamp apparatus, consists of a heat-retaining shade (30) capable of assembly with the body of the lamp via a one-touch method. This makes possible separate packaging and shipping of components, reducing the distribution cost dramatically, likewise the installation of the lamp in such locations as barns as a result of a simple assembly of the lamp body and shade.
The invention includes the following components: a socket (10) that provides the infrared bulb (60) electricity, a curved cooling back-plate (20) equipped with a projected cable outlet (22) that constitutes of more than one suspension hook on its edge, which covers the aforementioned socket to absorb heat generated from the socket and to lose heat to the surrounding air, a heat-retaining shade (30) that covers the light bulb to concentrate the heat to required area; the shade (30) and the cooling back-plate (20) are assembled through a one-touch manipulation.

## Description

### Technical field

This invention, concerning an infrared lamp apparatus, especially consists of a heat-retaining shade capable of assembly with the body of the lamp via a one-touch method. This makes possible separate packaging and shipping of components, reducing the distribution cost dramatically, likewise the installation of the lamp in such locations as barns as a result of a simple assembly of the lamp body and shade.

### Prior Art

Generally, infrared bulbs refer to bulbs radiating infrared rays, which emit thermal energy of approximately 200°C when lighted so it can heat small spaces. Accordingly, the infrared bulb is widely used in places like cattle sheds and small saunas in the bathroom.
The infrared bulb mentioned above is commonly joined electrically to a socket of a lamp apparatus, which is constituted of a socket, shade, and etc. In addition, to restrain the inside and outside of the socket from overheating, which is attributed by the self-generated heat from the socket and the heat conveyed from the bulb, a gap is formed between the socket and the heat-retaining shade to allows a small amount of ventilation.

However, previous infrared lamp apparatuses have been produced and supplied to customers as a whole; the body, which includes a socket, and a heat-retaining shade. As a result, the shade occupied much space when packaging the infrared lamp apparatus, and was very inefficient in terms of packaging space, contributing to a huge increase of distribution costs used in the storage and shipping of the lamp. Also, when the heat-retaining shade is damaged, circumstances force to replace the lamp apparatus as a whole, instead of just the damaged shade. Moreover, there is a high possibility for water to enter through the gap, which is formed between the socket and the shade to restrain the socket from overheating, and if the gap is made more narrow, then it would not function properly to cool down inside and outside of the socket.

### Technical Problem to be solved

This invention contrived to solve the above-mentioned problems, its aim to provide an infrared lamp apparatus with a simple assembly process. By a one-touch manipulation, the body that includes the socket, and the heat-retaining shade can be assembled to an end product, so that it can be installed in such places like barns.

Another goal for the invention is to reduce the distribution costs dramatically when providing the infrared lamp apparatus. Since the product end is assembled in such places like barns, the separate packaging, storage, and shipping of the components, being the body that includes the socket, and the heat-retaining shade, was made possible.

Again, since heat generated from the socket, and heat conveyed to the socket from the infrared bulb is easily emitted to the outside, another goal was to provide a safe infrared lamp apparatus to prevent accidents from the overheating of the body like the socket.

### Disclosure of invention

To achieve the goals mentioned above, the invention includes the following components with the following features: a socket that provides the infrared bulb electricity, a curved cooling back-plate equipped with a projected cable outlet that constitutes of more than one suspension hook on its limb, which covers the aforementioned socket to absorb heat generated from the socket and to lose heat to the surrounding air, a heat-retaining shade that covers the light bulb to concentrate the heat to required area, an assembly method to join the above shade and the cooling back-plate through a one-touch manipulation, a protective cap that is screwed on the cable outlet of the cooling back-plate so that an exterior electric cable is inserted, and constitutes of multiple preventive projections supporting the suspension hooks on the inside of the cable outlet, to prevent rotation in the direction that disassembles it from the cable outlet.

Below, the advisable example of this invention is explained in detail, with reference to the supplementary figures.

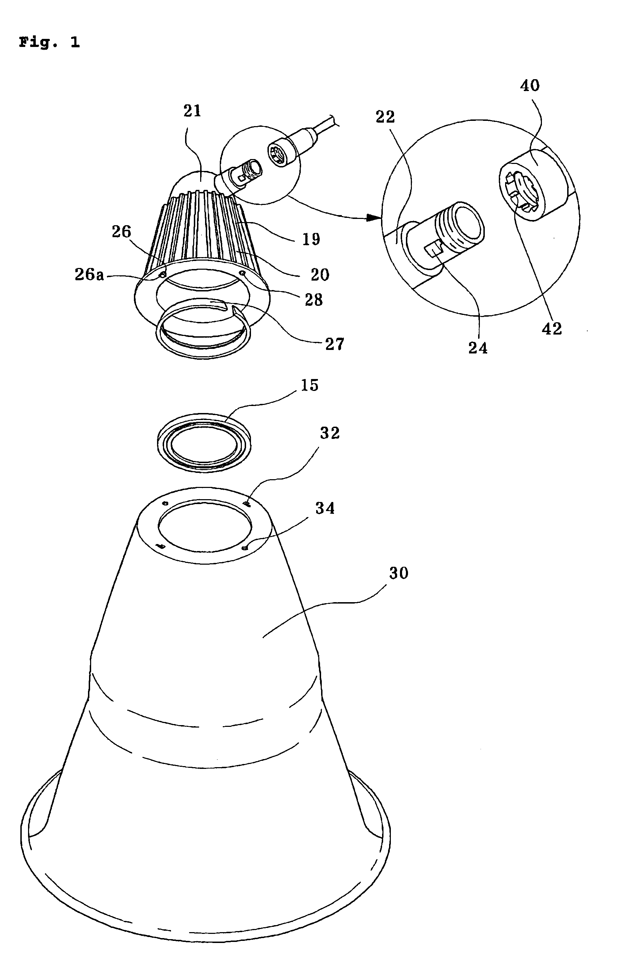

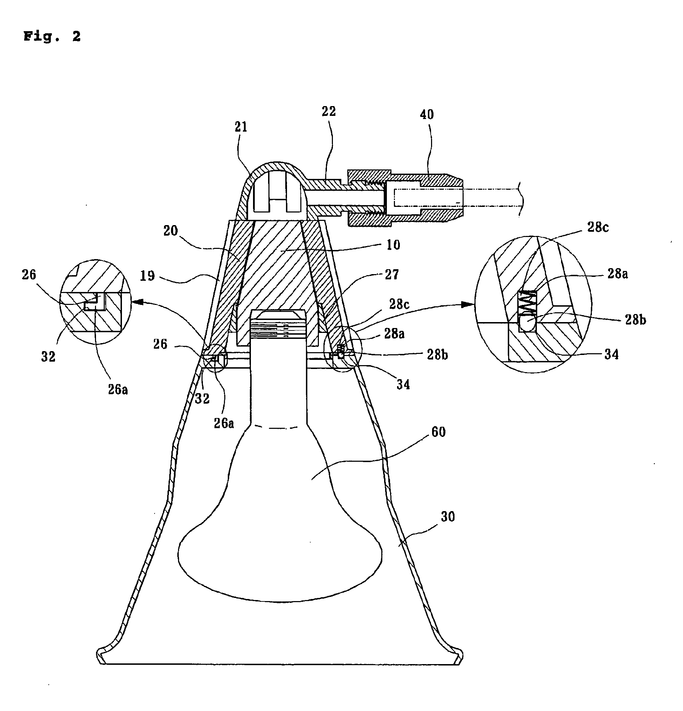

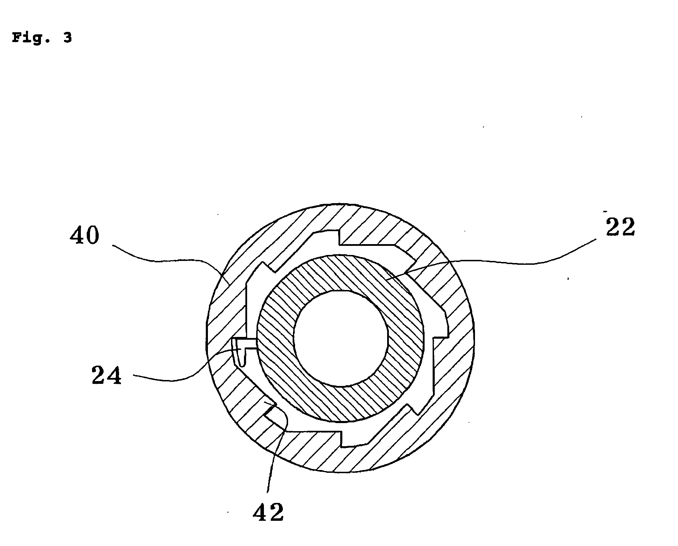

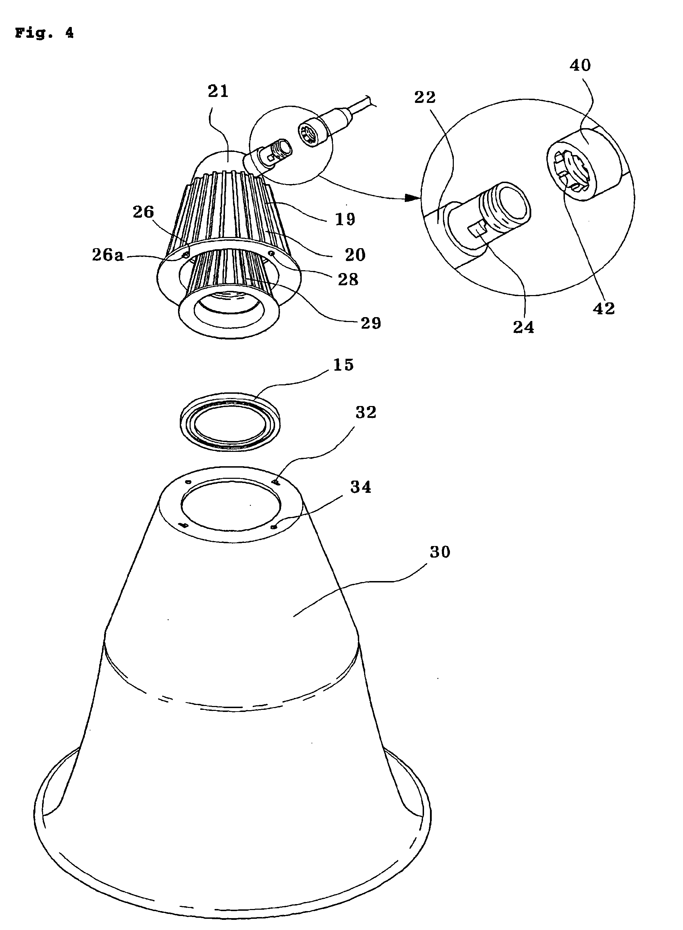

Figure 1 is a deal (disassembled) figure of all components of the infrared lamp apparatus; figure 2 is an assembled cross section figure of the infrared lamp apparatus that is executed accordingly to figure 1; figure 3 is a cross section figure of the assembled protective cap of the infrared lamp apparatus that is executed accordingly to figure 1; figure 4 is a deal (disassembled) figure of all components of the infrared lamp apparatus that uses a heat-conducting cap accordingly to a different example.

As illustrated in the figure, the infrared lamp apparatus of this invention is constituted of a socket (10), a curved cooling back-plate (20), a heat-retaining shade (30), an assembly method, and a protective cap (40).
The socket (10), which is detachable, is joined to the infrared bulb (60) to provide electricity, and the socket (10) is accordingly coupled to the power cable (70).

The cooling back-plate (20) holds the socket (10) in its inside to absorb the heat, and to be cooled by ventilation. When the infrared bulb is turned on, heat is generated from the socket (10) itself and also conveyed to the socket (10) from the infrared bulb (60), and thus the cooling back-plate is made with materials that have good heat-conductivity to prevent it from overheating. It is desirable for the cooling back-plate (20) to have a multitude of projected wings, so that it can improve its cooling effect by maximizing the ventilation area. Such wings (19) should be continuously laid out either horizontally, to allow a horizontal passage of air on the surface of the cooling back-plate (20), or vertically, to allow a vertical passage of air on the surface of the cooling back-plate. This will enable the air to flow smoothly on the surface of the cooling back-plate, and increase the cooling effect.

In addition, the cooling back-plate (20) is equipped with a cable outlet (22), and the power cable (70), which provides electricity to the infrared bulb (60) via the socket (10), is coupled with the cable outlet. It would be best for the cable outlet (22) to have multiple suspension hooks on its edge for a more firm assembly with the protective cap (40), which will be later mentioned with more details. Also, a plastic cover (21), which ought to have a cable outlet (22) and a suspension hook (24), is assembled to the upper part of the cooling back-plate (20).

The heat-retaining shade (20) should be assembled to the cooling back-plate (20) so that it covers the infrared bulb (60), and so that the heat from the infrared bulb (60) is concentrated to the area that needs to be heated. In other words, the heat-retaining shade (30) blocks the heat generated from the infrared bulb from spreading, and concentrates it to one area.

The heat-retaining shade (30) can be assembled to the cooling back-plate (20) by a rotating one-touch manipulation. This enables the separate moving of the heat-retaining shade (30) and the cooling back-plate (20), and the separate components can be easily assembled at the installation site.

Such assembly would be proper to be constituted of the rotation lock (26), the spring lock (28), and the primary and secondary locking grooves (32)(34).

The rotation lock (26) is prominently equipped on the underbody of the cooling back-plate (20), and has an enlarged head (26a).

More than two of the aforementioned spring lock (28) is created on the underbody of the cooling back-plate (20), and when a pressing force is applied the spring lock (28) is inserted inside the cooling back-plate (20), and when the pressing force is removed it projects to the outside of the cooling back-plate (20). The spring lock should have an inserting groove (28c) of a small depth on the underbody of the cooling back-plate, and the inserting groove (28c) should be equipped with a locking back-plate (28b) that has a spring (28a). It is needless to say it can be materialized in other forms.

Two or more primary locking grooves (32) are formed on the surface of the heat-retaining shade (30). These primary locking grooves (32) are to be attached to grooves with sizes corresponding to the head of the rotation lock (26) and to those with sizes corresponding to parts other than the head (26a) of the rotation lock (26). On this, after the head (26a) of the rotation lock (26) is inserted to the bigger groove of the primary locking groove (32) and rotated towards the smaller groove, the rotation lock (26) of the head (26a) gets supported by the rim of the smaller groove. In other words, the rotation lock (26) becomes securely locked to the primary locking groove (32). Two or more secondary locking grooves (34) are formed on the surface of the heat-retaining shade (30). These secondary locking grooves (34) insert the spring lock (28) to form a lock when the rotation lock (26) is rotated about the primary locking groove (32).

Due to the above combining method, since the either sides of the cooling back-plate (20) and the heat-retaining shade (30) are combined by the rotation lock (26) and the primary locking groove (32); and the spring lock (28) and the secondary locking groove (34) prevents further rotations, they are securely combined. The above process is performed by a one-touch rotating operation after placing the surface of the heat-retaining shade (30) in touch with the underbody of the cooling back-plate (20).

The protective cap (40) is connected to the cable outlet (22) of the cooling back-plate (20) via screw. The exterior power cable (70) is inserted through one side of the protective cap (40), and the inserted exterior power cable (70) is inserted to the cable outlet (22) of the cooling back-plate (20), thus getting in contact with the socket (10). Which means the protective cap (40) isolates and protects the insertion area of the cooling back-plate (20) where the exterior power cable (70) gets connected to the socket (10). To prevent the secured protective cap (40) from rotating towards the loosening direction, as illustrated in figure 3, the inner section of the protective cap (40) is made up of many preventive projections (42). These preventive projections (42) are hooked to the suspension hook (24) on the cable outlet (22) preventing the protective cap (40) from rotating towards the loosening directing. The heat-retaining ring (27) is installed between the bottom part of the cooling back-plate (28) and the socket (10) to allow the internal and external heat of the socket (10) to be transmitted to the cooling back-plate (20) with ease. However, for smooth insertion, cutting off a sufficient part of the heat-retaining ring (27) is encouraged.

Here, instead of the aforementioned heat-retaining ring (27), as illustrated in figure 4, the heat-conducting cap (29) is inserted. This heat-conducting cap (29) can absorb the internal and external heat of the socket (10) and transmit it to the cooling back-plate (20).

In other words, the aforementioned heat-conducting cap (29) is installed such that its inner and outer surface is in contact with the outer surface of the socket (10) and the inner surface of the cooling back-plate (20) respectively. At this point, it is more advisable to make the wing-projected structures to form on one side of the heat-conducting cap (29), and in contact with the inner surface of the cooling back-plate (20). That is, the heat-conducting cap (29) is a scaled-down version of the cooling back-plate (20), and according to this, an air-transferring vent is formed between the wing-projected structures of the heat-conducting cap (29) thus maximizing the cooling effect of the heat being transferred to the cooling back-plate via the wing-projected structure (19).

Also, installation of the rubber joint (15) is advised, as it cuts off the space between the surface of the infrared bulb (60) that enters the aforementioned socket (10) and the aforementioned cooling back-plate (20). Such rubber joint (15) is to be supported on the opened section where the insertion of the infrared bulb (60) into the cooling back-plate (20) takes place. Here, it is advised to install the rubber joint (15) in such a manner that it follows the opened section of the cooling back-plate (20) so that it is supported elastically as it is fabricated of elastic silicone. Due to the rubber joint (15), water or foreign substance springing from the bottom of the heat-retaining shade (30) is prevented from percolating into the socket (10).

And, a figure has been omitted in the illustration, but installation of the protecting mesh at the bottom of the protective cap is advised to prevent exterior objects from inflicting shock to the infrared bulb for safe installation.

Due to the aforementioned construction method, among the important parts that make up the lamp apparatus of the infrared bulb, the relatively voluminous parts such as the heat-retaining shade (30) can be separated and supplied together with parts such as the socket (10) and the cooling back-plate (20) that can be inferred as the main body. And they can all be assembled with ease and installed at the actual desired infrared bulb (60) installation location through the one-touch rotation assembly method to combine the heat-retaining shade (30) with the main body of the lamp apparatus.

The cooling back-plate (20) helps the internal and external heat of the socket (10) to be cooled by means of exterior air. In addition, the heat-conducting cap (50) placed between the socket (10) and cooling back-plate (20) plays the role of conveying the internal and external heat of the socket (10) to the cooling back-plate (20). This prevents electric accidents from taking place due to the internal and external overheating of the socket (10).

As it can be known from the aforementioned execution example, the lamp apparatus of the infrared bulb, according to this invention, enables the main body parts such as the socket, and the heat-retaining shade to be packed separately and transported. After - transportation, it can easily be assembled in the desired assembling location, for e.g. a barn. This dramatically decreases the storage and distribution costs, and not only does it enable the user to assemble the main body part and the heat-retaining shade with ease but is also efficient economically as it is possible to replace any single part of choice, making selective replacement possible. On top of it, the internal and external heat of the socket is well emitted to the exterior resulting in a better prevention of electric accidents.

### Brief description of figures

- Figure 1: is a deal (disassembled) figure of all components of the infrared lamp apparatus
- Figure 2: is assembled cross section figure of the infrared lamp apparatus that is executed accordingly to figure 1.
- Figure 3: is a cross section figure of the assembled protective cap of the infrared lamp apparatus executed accordingly to figure 1
- Figure 4: is a deal (disassembled) figure of all components of the infrared lamp apparatus that uses a heat-conducting cap accordingly to a different example.

### *Description of main components of figure*

- 10:: socket
- 20:: curved cooling back-plate
- 15:: rubber joint
- 21:: cover
- 22:: cable outlet
- 24:: suspension hook
- 26:: rotation lock
- 27:: heat-retaining ring
- 26a:: the head
- 28:: spring lock
- 28a:: spring
- 28b:: locking back-plate
- 28c:: inserting groove
- 29:: heat-conducting cap
- 30:: heat-retaining shade
- 32:: primary locking groove
- 34:: secondary locking groove
- 40:: protective cap
- 42:: preventive projection
- 60:: infrared bulb
- 70:: power cable

## Claims

1. Regarding the lamp apparatus of the infrared bulb, Socket whose infrared bulb is electrically taken care of;
The curved cooling back-plate having the aforementioned socket placed inside it so that the heat emanated from the socket is absorbed and lost to the external air, and the multiple hooks structured around the rim of the projected cable outlet;
The heat-retaining shade surrounding the aforementioned infrared bulb, which concentrates heat in the space where heating is required;
The lamp apparatus of the infrared bulb having the assembly method of assembling the aforementioned heat-retaining shade and the aforementioned cooling back-plate via one-touch operation; and the screw connecting mechanism involved when connecting the electric plug of the aforementioned cooling back-plate with exterior power cables as its special feature. Together with the protective cap made up of multiple projections supported by the hook in the cable outlet in order to prevent loosening rotation in the aforementioned cable outlet.

2. As stated in claim 1, the aforementioned assembly method is,
The lamp apparatus of the infrared bulb that includes two or more rotation locks projected on the either sides of the aforementioned cooling back-plate, forming a sectional head in the front part;
Possession of two or more spring locks in the bottom part of the cooling back-plate;
The two or more primary locking grooves joining the two different sized grooves on the surface of the heat-retaining shade, so that the head part of the rotation lock is rotated and supported on the rim of the groove after insertion;
And the two or more secondary locking grooves formed on the surface of the aforementioned heat-retaining shade so that the springy insertion of the aforementioned spring lock occurs when rotation takes place after positioning the aforementioned primary locking groove in a locked state.

3. As stated in claim 1, the lamp apparatus of the infrared bulb having a cooling back-plate with multiple wing-projected structures to increase the area in contact with external air as its special feature.

4. As stated in claim 3, the lamp apparatus of the infrared bulb having the aforementioned wing-projected structure, structured in a continuous horizontal manner to form horizontal air vents on the surface of the cooling back-plate, or in a continuous vertical manner to form vertical air vents on the surface of the cooling back-late as its special feature.

5. As stated in claim 1 or 3, the lamp apparatus of the infrared bulb having an additional heat-conducting cap installed between the exterior of the aforementioned socket and interior of the cooling back-plate touching each surfaces respectively to transmit the internal and external heat of the socket to the cooling back-plate as its special feature.

6. As stated in claim 5, the lamp apparatus of the infrared bulb having the aforementioned heat-conducting cap composed of many wing-projected structures on the side in contact with the aforementioned cooling back-plate as its special feature.

7. As stated in claim 5 or 6, the lamp apparatus of the infrared bulb having the aforementioned heat-conducting cap that has a heat-retaining ring installed pressurized in the lower internal section of the aforementioned cooling back-plate as its special feature.

8. As stated in claim 1, the lamp apparatus of the infrared bulb having an additional rubber joint used to cut off the space between the aforementioned infrared bulb in the socket and the aforementioned cooling back-plate by supporting it on the opened section where the aforementioned infrared bulb of the cooling back-plate is inserted as its special feature.

9. As stated in claim 8, the lamp apparatus of the infrared bulb having the aforementioned rubber joint made from extruded silicone as its special feature.
